# EUROPÄISCHE PATENTSCHRIFT

(11) **EP 1 775 586 B1**
(45) Veröffentlichungstag und Bekanntmachung des Hinweises auf die Patenterteilung: **14.12.2011**
(21) Anmeldenummer: 05022618.2
(22) Anmeldetag: 17.10.2005
(51) Int. Cl.: G01N 33/487, C12M 1/34

(54) **Verfahren zur elektrophysiologischen Untersuchung einer einen Ionenkanal umfassenden Membran**
Method for electrophysiological investigation of a membran having an ion channel
Procédé d'examen électrophysiologique d'une membrane comprenant un canal ionique

(43) Veröffentlichungstag der Anmeldung: 18.04.2007
(73) Patentinhaber: Nanion Technologies GmbH, 80336 München (DE)
(72) Erfinder: Fertig, Niels, Dr., 80335 München (DE); Brüggemann, Andrea, 81667 München (DE)
(74) Vertreter: Grünecker, Kinkeldey, Stockmair & Schwanhäusser

(56) Entgegenhaltungen:
- LUKYANETZ E A ET AL: "Different types of calcium channels and secretion from bovine chromaffin cells" EUROPEAN JOURNAL OF NEUROSCIENCE, Bd. 11, Nr. 8, August 1999 (1999-08), Seiten 2865-2873, XP002368343 ISSN: 0953-816X
- FRINGS S ET AL: "SINGLE-CHANNEL RECORDINGS FROM THE APICAL MEMBRANE OF THE TOAD URINARY BLADDER EPITHELIAL CELL" JOURNAL OF MEMBRANE BIOLOGY, Bd. 106, Nr. 2, 1988, Seiten 157-172, XP002371309 ISSN: 0022-2631
- HAMILL O P ET AL: "IMPROVED PATCH CLAMP TECHNIQUES FOR HIGH RESOLUTION CURRENT RECORDING FROM CELLS AND CELL-FREE MEMBRANE PATCHES" PFLUEGERS ARCHIV EUROPEAN JOURNAL OF PHYSIOLOGY, Bd. 391, Nr. 2, 1981, Seiten 85-100, XP000196663 ISSN: 0031-6768
- SUAREZ-ISLA B A ET AL: "SINGLE CALCIUM CHANNELS IN NATIVE SARCOPLASMIC RETICULUM MEMBRANES FROM SKELETAL MUSCLE" PROCEEDINGS OF THE NATIONAL ACADEMY OF SCIENCES OF THE UNITED STATES OF AMERICA, Bd. 83, Nr. 20, 1986, Seiten 7741-7745, XP002368345 ISSN: 0027-8424
- NAGANO TOMOO ET AL: "Evidence for norepinephrine-activated Ca2+ permeable channels in guinea-pig hepatocytes using a patch clamp technique" JOURNAL OF NIPPON MEDICAL SCHOOL, Bd. 66, Nr. 2, April 1999 (1999-04), Seiten 127-133, XP002368346 ISSN: 0048-0444
- DURROUX T ET AL: "BACKGROUND CALCIUM PERMEABLE CHANNELS IN GLOMERULOSA CELLS FROM ADRENAL GLAND" JOURNAL OF MEMBRANE BIOLOGY, Bd. 129, Nr. 2, 1992, Seiten 145-153, XP002368347 ISSN: 0022-2631
- FERTIG NIELS ET AL: "Whole cell patch clamp recording performed on a planar glass chip" BIOPHYSICAL JOURNAL, Bd. 82, Nr. 6, Juni 2002 (2002-06), Seiten 3056-3062, XP001206251 ISSN: 0006-3495

## Beschreibung

Die Erfindung betrifft ein Verfahren zur elektrophysiologischen Untersuchung einer einen Ionenkanal umfassenden Membran.

Zellmembranen (oder auch künstliche Lipidmembranen) weisen lonenkanäle auf, d.h. Transmembranproteine mit Poren, die einen Stromfluss durch die Membran ermöglichen. Die Funktionsweise derartiger lonenkanäle kann mit Methoden der Elektrophysiologie, insbesondere mit der Patch-Clamp-Technik, untersucht werden. Auf diese Weise lassen sich beispielsweise Öffnungs- und Schließmechanismen der lonenkanäle analysieren.

Bei dem herkömmlichen Patch-Clamp-Verfahren werden sogenannte Patch-Clamp-Pipetten verwendet, deren Öffnungsdurchmesser an der Spitze etwa 1 µm beträgt. In dem Pipettenschaft sind eine Elektrolytlösung (intrazelluläre Lösung) und eine Elektrode vorgesehen. An die Öffnung einer mit Elektrolytlösung gefüllten Pipette wird ein Membranfleck durch einen Unterdruck angesaugt, so dass sich zwischen der Membran und dem Pipettenglas ein enger Kontakt bildet. Zwischen dem Innern der Pipette und der Außenlösung (extrazellulären Lösung) sollte nun ein hoher Abdichtwiderstand in einer Größenordnung von mehr als einem GΩ entstehen. Auf diese Weise lassen sich Ionenkanalströme durch den angesaugten Membranfleck messen.

Dieses herkömmliche Verfahren ist jedoch nicht für Hochdurchsatzuntersuchungen geeignet. Zu einem solchen Zweck sind jedoch Biochips mit einem Substrat bekannt, in welchem ein Array von Öffnungen zur Aufnahme von Zellmembranen vorgesehen sind. Eine derartige Vorrichtung ist beispielsweise aus der WO 02/066596 bekannt.

Herkömmlicherweise verwendete Elektrolytlösungen für die Innen- und die Außenlösung sind beispielsweise in A. Ludwig et al., "A family of hyperpolarization-activated mammalian cation channels", Nature, 1998, Vol. 393, 587 - 591, A. Brueggemann et al., "lon Channel Drug Discovery and Research: The Automated Nano-Patch-Clamp Technology", Current Drug Discovery Technologies, 2004, 1, 91-96 oder D. Prawitt et al., "TRPM5 is a transient Ca2+-activated cation channel responding to rapid changes in [Ca2+]i" PNAS, 2003, Vol. 100, No. 25, 15166-15171 beschrieben.

Bei den genannten Vorrichtungen, die zur automatisierten Durchführung von Patch-Clamp-Verfahren vorgesehen sind, tritt das Problem auf, dass bei der Verwendung herkömmlicher Elektrolytlösungen nach dem Ansaugen des Membranflecks nicht immer ein ausreichend hoher Abdichtwiderstand in der Größenordnung von mehr als einem GΩ erhalten wird.

E. A. Lukyanetz et al., "Different types of calcium channels and secretion from bovine chromaffin cells", Eur. J. Neuroscience, Vol. 11, Seiten 2865 bis 2873, 1999, beschreibt ein herkömmliches Patch-Clamp-Vertahren, bei dem die Badlösung 60 mM CaCl₂ und die intrazelluläre Lösung 64 mM CsCl enthalten.

O. P. Hamill et al., "Improved Patch-Clamp Techniques for High-Resolution Current Recording from Cells and Cell-Free Membrane Patches", Pflügers-Archiv Eur. J. Physiology, Vol. 391, Seiten 85 bis 100, 1981, beschreibt ein herkömmliches Patch-Clamp-Vertahren.

N. Fertig et al., "Whole Cell Patch Clamp Recording Performed on a Planar Glass Chip", Biophysical Journal, Vol. 82, Seiten 3056 bis 3062, 2002, beschreibt ein Patch-Clamp-Verfahren mittels eines perforierten Quarzchips.

Die Aufgabe der Erfindung besteht darin, den Abdichtwiderstand bei elektrophysiologischen Untersuchungen zu verbessern.

Diese Aufgabe wird gelöst durch ein Verfahren nach Anspruch 1.

Dafür wird eine Kollektion zum Ausbilden eines hohen elektrischen Widerstands bei einer elektrophysiologischen Untersuchung einer einen lonenkanal umfassenden Membran bereitgestellt, umfassend eine erste Elektrolytlösung und eine zweite Elektrolytlösung, wobei die erste Elektrolytlösung 20-140 mM divalente Kationen eines ersten Elements und die zweite Elektrolytlösung 20 - 200 mM monovalente Anionen eines zweiten Elements umfasst.

Es hat sich überraschenderweise herausgestellt, dass mit einer derartigen Kombination von Elektrolytlösungen bei der Durchführung eines Patch-Clamp-Verfahrens mit einem Biochip eine signifikant verbesserte Bildung eines Abdichtwiderstands erhalten wird. Dies tritt auf, wenn die erste Elektrolytlösung als extrazelluläre Lösung (Außenlösung) und die zweite Elektrolytlösung als intrazelluläre Lösung (Innenlösung) eingesetzt wird.

Die erste Elektrolytlösung kann 30 - 80 mM, insbesondere 30 - 50 mM, divalente Kationen des ersten Elements umfassen. Alternativ oder zugieich kann die zweite Elektrolytlösung 50-150 mM, insbesondere 60 - 140 mM, monovalente Anionen des zweiten Elements umfassen. Bei der Verwendung von Elektrolytlösungen mit diesen Stoffmengenbereichen ergibt sich eine weiter verbesserte Widerstandsbildung.

Das erste Element kann Kalzium (Ca) oder Magnesium (Mg) und/oder das zweite Element kann Fluor (F) oder Chlor (Cl) sein. Insbesondere kann die erste Elektrolytlösung Ca-Ionen und die zweite Elektrolytlösung F-Ionen in den zuvor genannten Stoffmengen umfassen.

Die divalenten Kationen des ersten Elements können Kationen eines Chlorid-Salzes sein. Insbesondere kann in der ersten Elektrolytlösung CaCl₂ in der genannten Stoffmenge gelöst sein.

Die monovalenten Anionen des zweiten Elements können Fluorid-Anionen sein. Insbesondere können in der zweiten Elektrolytlösung KF oder CsF in den genannten Stoffmengen gelöst sein. Beispielsweise kann in der ersten Elektrolytlösung CaCl₂ und in der zweiten Elektrolytlösung KF gelöst sein. Alternativ können die monovalenten Anionen des zweiten Elements Chlorid-Anionen sein. So kann beispielsweise in der zweiten Elektrolytlösung NaCl in der genannten Stoffmenge gelöst sein.

Die Kationen und/oder die Anionen können in einer physiologischen Salzlösung, beispielsweise einer Ringerlösung, gelöst sein. Dies erlaubt eine Untersuchung von Zellmembranen in ihrer natürlichen Umgebung.

Die erste und/oder die zweite Elektrolytlösung kann einen pH-Wert zwischen 7 und 7,5 und/oder eine Osmolarität zwischen 200 und 400 mOsm, insbesondere zwischen 240 und 330 mOsm, aufweisen.

Insbesondere kann die Kollektion für das Durchführen eines Patch-Clamp-Verfahrens von Erythrozyten, Primärkulturzellen oder Kardiomyozyten verwendet werden. Es hat sich herausgestellt, dass mit der Kombination von Elektrolytlösungen auch Patch-Clamp-Untersuchungen von Zellen, wie Erythrozyten, isolierte Zellen/Primärkulturzellen oder Kardiomyozyten, durchgeführt werden können.

Bei den genannten Verwendungen wird die erste Elektrolytlösung als extrazelluläre Lösung und die zweite Elektrolytlösung als intrazelluläre Lösung eingesetzt.

Die Erfindung ist ein Verfahren zur elektrophysiologischen Untersuchung einer einen lonenkanal umfassenden Membran, insbesondere einer Zellmembran, mit den Schritten:
Bereitstellen einer Trennwand mit wenigstens einer Öffnung zur Aufnahme der Membran,
Bereitstellen einer der zuvor beschriebenen Kollektionen,
Positionieren der Membran an einer der wenigstens einen Öffnung auf einer ersten Seite der Trennwand, so dass die Membran den Öffnungsrand berührt, wobei auf der ersten Seite der Trennwand die erste Elektrolytlösung und auf der zweiten Seite der Trennwand die zweite Elektrolytlösung vorgesehen ist,
Bestimmen des Stroms durch oder der Spannung über den Ionenkanal.

Erfindungsgemäß wird die erste Elektrolytlösung nach dem Positionieren der Membran, und vor dem Schritt des Bestimmens, hinzugegeben.

Es ist also beispielsweise möglich, zu untersuchende Zellen in ihrem ursprünglichen Nährmedium oder einer Elektrolytlösung bereitzustellen und an der Öffnung der Trennwand zu positionieren. Letzteres kann beispielsweise durch Anlegen eines Unterdrucks durch die Öffnung und entsprechendes Ansaugen der Zelle geschehen. Erst danach wird die erste Elektrolytlösung hinzugegeben , so dass der Abdichtwiderstand stark ansteigt.

Vor dem Schritt des Bestimmens kann ein Spülen durchgeführt werden, um die erste Elektrolytlösung im Wesentlichen zu entfemen.

Es hat sich überraschenderweise herausgestellt, dass der mittels der Kombination der erfindungsgemäßen ersten und zweiten Elektrolytlösung erreichte, sehr hohe Abdichtwiderstand auch im Wesentlichen erhalten bleibt, wenn anschließend ein Spülen vorgenommen wird. Dies bedeutet, dass die Untersuchung der Membran anschließend auch mit einer anderen, beliebigen Lösung durchgeführt werden kann, ohne dass sich der Abdichtwiderstand signifikant verändert.

Als Trennwand kann ein perforiertes Substrat, insbesondere aus Glas oder einem Halbleitermaterial, bereitgestellt werden. Dadurch lässt sich das Verfahren insbesondere mit einem Biochip mit einem derartigen Substrat in automatisierter Weise durchführen, was Hochdurchsatzuntersuchungen ermöglicht.

Insbesondere können bei den zuvor beschriebenen Verfahren Öffnungen mit einem Durchmesser von 0,1 -10 µm bereitgestellt werden.

Weitere Vorteile und Merkmale werden im Folgenden an Hand der Figuren beschrieben.

Dabei zeigt
- Figur 1: eine Messsonde zum Durchführen einer elektrophysiologischen Unter-suchung und
- Figur 2: eine Grafik zur Illustration des Ansteigens des Abdichtwiderstands.

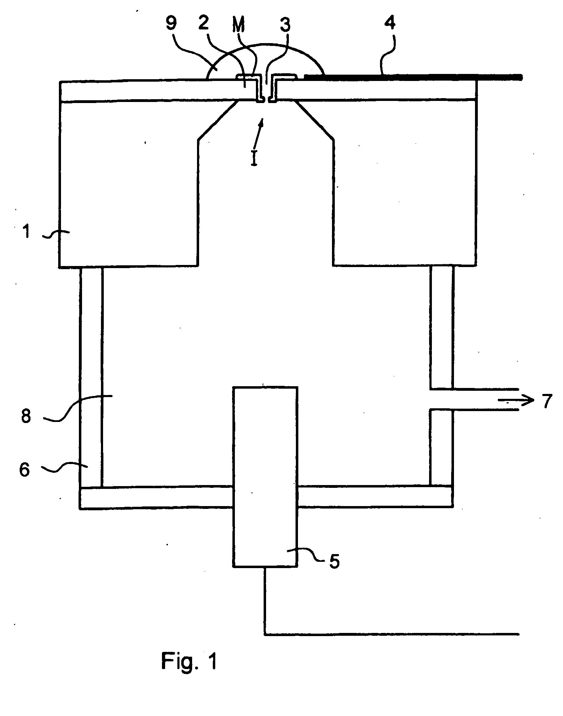

Die in Figur 1 gezeigte Messsonde umfasst ein Substrat mit einem Basisabschnitt 1 und einem Fensterabschnitt 2, in dem eine Öffnung 3 ausgebildet ist. Der Basisabschnitt kann beispielsweise aus Quarz oder einem Halbleitermaterial, beispielsweise (100)-Si, bestehen.

Der Fensterabschnitt 2 ist in einer isolierenden Schicht ausgebildet, die beispielsweise aus Glas besteht. Die Herstellung eines derartigen Substrats mit einem Basisabschnitt und einem Fensterabschnitt wird beispielsweise in der WO 02/066596 beschrieben.

Auf dem Substrat ist eine erste Elektrode 4 angeordnet. Alternativ kann diese Elektrode auch einfach in die Lösung gehalten werden ohne unmittelbar auf dem Substrat angeordnet zu sein. Eine zweite Elektrode 5 befindet sich unterhalb des Substrats. Die Elektroden können beispielsweise aus Ag/AgCl bestehen.

Über eine Haltevorrichtung 6 wird ein Hohlraum gebildet, der mit der Apertur 3 eine Öffnung besitzt. Die Messsonde umfasst weiterhin eine Einrichtung zum Erzeugen von Unterdruck in der Haltevorrichtung, wie durch das Bezugszeichen 7 angedeutet ist.

In den Hohlraum, d.h. unterhalb des Chips, wird eine intrazelluläre Lösung 8, auf den Chip eine extrazelluläre Lösung 9 gegeben.

Herkömmliche Lösungen, die als intrazelluläre und extrazelluläre Lösungen verwendet werden, sind beispielsweise in dem oben genannten Artikel von A. Ludwig *et al.* beschrieben. So kann die extrazelluläre Lösung beispielsweise die folgende Zusammensetzung haben: 110 mM NaCl, 0,5 mM MgCl₂, 1,8 mM CaCl₂, 5 mM HEPES, 30 mM KCl, mit NaOH auf pH 7,4 eingestellt. Eine intrazelluläre Lösung kann die Zusammensetzung haben: 130 mM KCI, 10 mM NaCl, 0,5 mM MgCl₂, 10 mM EGTA, 10 mM HEPES, mit KOH auf pH 7,4 eingestellt.

Bei der Durchführung einer elektrophysiologischen Untersuchung gemäß der vorliegenden Erfindung kann beispielsweise wie folgt vorgegangen werden. Zunächst wird der Hohlraum mit einer erfindungsgemäßen zweiten Elektrolytlösung als intrazelluläre Lösung gefüllt. Eine geeignete intrazelluläre Lösung kann beispielsweise die Zusammensetzung haben: 10 mM KCI, 135 mM KF, 10 mM NaCl, 2 mM MgCl₂, 10 mM EGTA, 10 mM HEPES, pH 7,2, 320 mOsm.

Auf den Chip wird zunächst eine herkömmliche extrazelluläre Lösung gegeben, wie sie beispielsweise oben im Zusammenhang mit den zitierten Artikeln beschrieben wurde. Anschließend werden die zu untersuchenden Zellen oder Membranen in einer herkömmlichen extrazellulären Lösung hinzugegeben. In Figur 1 ist eine derartige Membran M mit einem lonenkanal I angedeutet.

Durch Anlegen eines Unterdrucks wird die Membran bzw. die Zelle in die Öffnung 3 gesaugt, was sich in einer Erhöhung des Abdichtwiderstands bemerkbar macht.

Anschließend wird auf den Chip eine erfindungsgemäße erste Elektrolytlösung als extrazelluläre Lösung hinzugegeben, so dass nun die erfindungsgemäße Kombination von erster und zweiter Elektrolytlösung vorliegt. Die hat einen signifikanten Anstieg des Abdichtwiderstands zur Folge. Die hinzugegebene Elektrolytlösung kann die Zusammensetzung haben: 105 mM NaCl, 4,5 mM KCI, 1 mM MgCl₂, 40 mM CaC1₂, 10 mM HEPES, 5 mM Glukose, pH 7,4, 320 mOsm.

Durch die beispielhaft genannte Kombination von 40 mM divalenten Ca-lonen in der extrazellulären Lösung und 135 mM monovalenten F-lonen in der intrazellulären Lösung ergibt sich eine große Verbesserung des elektrischen Widerstands über die Öffnung. Damit lassen sich nun Patch-Clamp-Untersuchungen insbesondere mit einem sehr guten Signal-zu-Rausch-Verhältnis durchführen.

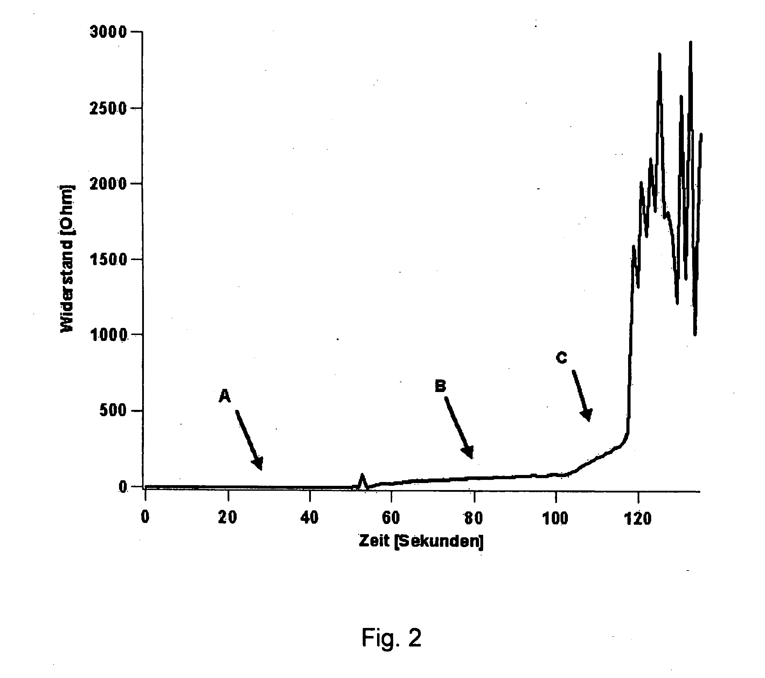

Die Veränderung des Abdichtwiderstands über die Zeit ist in Figur 2 dargestellt. Zu Beginn, solange auf beiden Seiten der Öffnung lediglich die Lösungen vorhanden sind, liegt der Widerstand der Öffnung in der Größenordnung von etwa 2-3 MΩ (Abschnitt A). Nach Zugeben der Zellen steigt der Widerstand auf etwa 20 - 50 MΩ an, sobald eine Membran an der Öffnung positioniert ist. Dies ist in Abschnitt B in der Grafik der Figur 2 zu erkennen. Bis hier weist zwar die intrazelluläre Lösung eine erfindungsgemäße Zusammensetzung auf, bei der extrazellulären Lösung handelt es sich jedoch um eine herkömmliche Lösung, wie sie beispielsweise zuvor beschrieben wurde.

Nach Zugabe der erfindungsgemäßen Lösung (Abschnitt C) steigt der Abdichtwiderstand jedoch auf über 1 GΩ an. Daraus ist klar zu ersehen, dass die Erhöhung des Abdichtwiderstands auf die Kombination der beiden erfindungsgemäßen Lösungen zurückzuführen ist.

Es versteht sich, dass das zuvor beschriebene, beispielhafte Verfahren auch modifiziert werden kann.

So kann beispielsweise die extrazelluläre erfindungsgemäße Lösung nach Erreichen des hohen Abdichtwiderstands wieder gespült werden, so dass die anschließenden Messungen mit anderen herkömmlichen extrazellulären Lösungen durchgeführt werden können. Es zeigt sich, dass auch in diesem Fall der hohe Abdichtwiderstand erhalten bleibt.

Weiterhin beschränkt sich der überraschende Effekt nicht auf die spezifischen, zuvor beispielhaft genannten Lösungszusammensetzungen. So können beispielsweise divalente Ca- oder Mg-Ionen innerhalb der oben angegebenen Stoffmengenbereichen in einer physiologischen Salzlösung, beispielsweise wie der in Prawitt *et al.* genannten Ringerlösung, als CaCl₂ oder MgCl₂ gelöst sein. Entsprechend kann auch Fluorid oder Chlorid, solange es in den angegebenen Stoffmengenbereichen liegt, in einer physiologischen Salzlösung gelöst bereitgestellt werden.

Weiterhin beschränkt sich die Erfindung nicht auf die Anwendung bei der in Figur 1 gezeigten Messsonde. Die Kollektion von erster Elektrolytlösung und zweiter Elektrolytlösung mit den angegebenen Stoffmengenbereichen an divalenten Kationen und monovalenten Anionen kann beispielsweise auch bei Hochdurchsatz-Biochips mit Arrays mit einer Vielzahl von Öffnungen eingesetzt werden.

## Patentansprüche

1. Verfahren zur elektrophysiologischen Untersuchung einer einen lonenkanal umfassenden Membran (M), insbesondere einer Zellmembran, mit den Schritten:
Bereitstellen einer Trennwand (2) mit wenigstens einer Öffnung (3) zur Aufnahme der Membran,
Bereitstellen einer ersten Elektrolytlösung (8) und einer zweiten Elektrolytlösung (9),
wobei die erste Elektrolytlösung 20 - 140 mM divalente Kationen eines ersten Elements und die zweite Elektrolytlösung 20 - 200 mM monovalente Anionen eines zweiten Elements umfasst,
Positionieren der Membran an einer der wenigstens einen Öffnung auf einer ersten Seite der Trennwand, so dass die Membran den Öffnungsrand berührt, wobei auf der ersten Seite der Trennwand die erste Elektrolytlösung und auf der zweiten Seite der Trennwand die zweite Elektrolytlösung vorgesehen ist, wobei die erste Elektrolylösung nach dem Positionieren der Membran hinzugegeben wird,
Bestimmen des Stroms durch oder der Spannung über den lonenkanal.

2. Verfahren nach Anspruch 1, wobei vor dem Schritt des Bestimmens ein Spülen durchgeführt wird, um die erste Elektrolytlösung im Wesentlichen zu entfernen.

3. Verfahren nach einem der Ansprüche 1 und 2, wobei als Trennwand ein perforiertes Substrat, insbesondere aus Glas, bereitgestellt wird.

4. Verfahren nach einem der Ansprüche 1 bis 3, wobei Öffnungen mit einem Durchmesser von 0,1 - 10 µm bereitgestellt werden.

5. Verfahren nach einem der vorangegangenen Ansprüche, wobei die erste Elektrolytlösung 30 - 80 mM, insbesondere 30 - 50 mM, divalente Kationen des ersten Elements und/oder die zweite Elektrolytlösung 50 - 150 mM, insbesondere 60 - 140 mM, monovalente Anionen des zweiten Elements umfasst.

6. Verfahren nach einem der vorangegangenen Ansprüche, wobei das erste Element Ca oder Mg und/oder das zweite Element F oder Cl ist.

7. Verfahren nach einem der vorangegangenen Ansprüche, wobei die divalenten Kationen des ersten Elements Kationen eines Chlorid-Salzes sind.

8. Verfahren nach einem der vorangegangenen Ansprüche, wobei die monovalenten Anionen des zweiten Elements Fluorid-Anionen sind.

9. Verfahren nach einem der vorangegangenen Ansprüche, wobei die Kationen und/oder die Anionen in einer physiologischen Salzlösung gelöst sind.

10. Verfahren nach einem der vorangegangenen Ansprüche, wobei die erste und/oder die zweite Elektrolytlösung einen pH-Wert zwischen 7 und 7,5 und/oder eine Osmolarität zwischen 200 und 400 mOsm, insbesondere zwischen 240 und 330 mOsm, aufweisen.

11. Verfahren nach einem der vorangegangenen Ansprüche, wobei die Membran eine Membran von Erythrozyten, Primärkulturzellen oder Kardiomyozyten ist.

12. Verfahren nach einem der vorangegangenen Ansprüche, wobei die erste Elektrolytlösung als extrazelluläre Lösung und die zweite Elektrolytlösung als intrazelluläre Lösung eingesetzt wird.

## Claims

1. Method for the electrophysiological examination of a membrane (M) comprising an ion channel, in particular of a cell membrane, comprising the steps of:
providing a dividing wall (2) having at least one aperture (3) to receive the membrane,
providing a first electrolytic solution (8) and a second electrolytic solution (9), the first electrolytic solution comprising 20 to 140 mM divalent cations of a first element and the second electrolytic solution comprising 20 to 200 mM monovalent anions of a second element,
positioning the membrane on one of the at least one apertures on a first side of the dividing wall, in such a way that the membrane touches the edge of the aperture, the first electrolytic solution being provided on the first side of the dividing wall and the second electrolytic solution being provided on the second side of the dividing wall, the first electrolytic solution being added after the positioning of the membrane,
determining the current through or the voltage across the ion channel.

2. Method according to claim 1, wherein rinsing is carried out prior to the determination step in order to substantially remove the first electrolytic solution.

3. Method according to either claim 1 or claim 2, wherein a perforated substrate, in particular made of glass, is provided as a dividing wall.

4. Method according to any one of claims 1 to 3, wherein apertures having a diameter of 0.1 to 10 µm are provided.

5. Method according to any one of the preceding claims, wherein the first electrolytic solution comprises 30 to 80 mM, in particular 30 to 50 mM, divalent cations of the first element and/or the second electrolytic solution comprises 50 to 150 mM, in particular 60 to 140 mM, monovalent anions of the second element.

6. Method according to any one of the preceding claims, wherein the first element is Ca or Mg and/or the second element is F or Cl.

7. Method according to any one of the preceding claims, wherein the divalent cations of the first element are cations of a chloride salt.

8. Method according to any one of the preceding claims, wherein the monovalent anions of the second element are fluoride anions.

9. Method according to any one of the preceding claims, wherein the cations and/or the anions are dissolved in a physiological saline solution.

10. Method according to any one of the preceding claims, wherein the first and/or the second electrolytic solution have a pH between 7 and 7.5 and/or an osmolarity between 200 and 400 mOsm, in particular between 240 and 330 mOsm.

11. Method according to any one of the preceding claims, wherein the membrane is a membrane of erythrocytes, primary culture cells or cardiomyocytes.

12. Method according to any one of the preceding claims, wherein the first electrolytic solution is used as an extracellular solution and the second electrolytic solution being used as an intracellular solution.

## Revendications

1. Procédé pour effectuer l'étude électrophysiologique d'une membrane (M) comportant un canal ionique, en particulier une membrane cellulaire, comprenant les étapes suivantes :
la préparation d'une paroi de séparation (2) avec au moins une ouverture (3) pour recevoir la membrane,
la préparation d'une première solution électrolytique (8) et d'une seconde solution électrolytique (9), dans lequel la première solution électrolytique comprend 20 à 140 mM de cations divalents d'un premier élément et la seconde solution électrolytique comprend 20 à 200 mM d'anions monovalents d'un second élément,
le positionnement de la membrane sur la au moins une ouverture sur un premier côté de la paroi de séparation, de sorte que la membrane touche le bord de l'ouverture, dans lequel sur le premier côté de la paroi de séparation, il est prévu la première solution électrolytique et sur le second côté de la paroi de séparation, il est prévu la seconde solution électrolytique, la première solution électrolytique étant ajoutée après que la membrane a été positionnée,
la détermination du courant dans le canal ionique ou de la tension aux bornes du canal ionique.

2. Procédé selon la revendication 1, dans lequel avant l'étape de détermination, on effectue un rinçage pour éliminer sensiblement la première solution électrolytique.

3. Procédé selon l'une quelconque des revendications 1 et 2, dans lequel, comme paroi de séparation, on fournit un substrat perforé, en particulier en verre.

4. Procédé selon l'une quelconque des revendications 1 à 3, dans lequel on fournit des ouvertures ayant un diamètre de 0,1 à 10 µm.

5. Procédé selon l'une quelconque des revendications précédentes, dans lequel la première solution électrolytique comprend 30 à 80 mM, en particulier 30 à 50 mM, de cations divalents du premier élément et/ou la seconde solution électrolytique comprend 50 à 150 mM, en particulier 60 à 140 mM, d'anions monovalents du second élément.

6. Procédé selon l'une quelconque des revendications précédentes, dans lequel le premier élément est l'élément Ca ou Mg et/ou le second élément est l'élément F ou CI.

7. Procédé selon l'une quelconque des revendications précédentes, dans lequel les cations divalents du premier élément sont des cations d'un sel de chlorure.

8. Procédé selon l'une quelconque des revendications précédentes, dans lequel les anions monovalents du second élément sont des anions de fluorure.

9. Procédé selon l'une quelconque des revendications précédentes, dans lequel les cations et/ou les anions sont dissous dans une solution de sel physiologique.

10. Procédé selon l'une quelconque des revendications précédentes, dans lequel la première solution électrolytique et/ou la seconde solution électrolytique présente(nt) une valeur de pH comprise entre 7 et 7,5 et/ou une osmolarité comprise entre 200 et 400 mOsm, en particulier entre 240 et 330 mOsm.

11. Procédé selon l'une quelconque des revendications précédentes, dans lequel la membrane est une membrane d'érythrocytes, de cellules de culture primaire ou de cardiomyocytes.

12. Procédé selon l'une quelconque des revendications précédentes, dans lequel la première solution électrolytique est utilisée comme solution extracellulaire, tandis que la seconde solution électrolytique est utilisée comme solution intracellulaire.
